**Europäisches Patentamt**

**European Patent Office**

**Office européen des brevets**

(19)

(11) Numéro de publication : **0 179 685**
**B1**

(12)

# FASCICULE DE BREVET EUROPÉEN

(45) Date de publication du fascicule du brevet : **28.09.88**

(21) Numéro de dépôt : **85401819.9**

(22) Date de dépôt : **19.09.85**

(51) Int. Cl.⁴ : **C 07 C 67/11**, C 07 C 69/145, C 07 C 69/63// C07C17/02, C07C21/04

(54) Procédé de préparation d'esters allyliques tertiaires éventuellement halogénés.

(30) Priorité : **20.09.84 FR 8414427**
**15.03.85 FR 8503841**

(43) Date de publication de la demande :
**30.04.86 Bulletin 86/18**

(45) Mention de la délivrance du brevet :
**28.09.88 Bulletin 88/39**

(84) Etats contractants désignés :
**AT BE CH DE FR GB IT LI LU NL SE**

(56) Documents cités :
**US-A- 3 062 874**
**US-A- 3 076 839**

(73) Titulaire : **RHONE-POULENC SANTE**
**Les Miroirs 18 Avenue d'Alsace**
**F-92400 Courbevoie Cédex (FR)**

(72) Inventeur : **Mulhauser, Michel**
**Immeuble "Frênes 4" Résidence Charrière Blanche**
**F-69130 Ecully (FR)**

(74) Mandataire : **Pilard, Jacques et al**
**RHONE-POULENC RECHERCHES Service Brevets**
**Pharma 25, Quai Paul Doumer**
**F-92408 Courbevoie Cedex (FR)**

## Description

La présente invention concerne un nouveau procédé de préparation d'un ester allylique tertiaire éventuellement halogéné par action d'un sel de métal alcalin d'un acide carboxylique sur un halogénure allylique primaire et/ou tertiaire éventuellement halogéné ou leurs mélanges en présence d'un catalyseur constitué d'un sel cuivreux associé à un sel d'ammonium quaternaire ou à un sel de phosphonium.

La littérature enseigne différents procédés de préparation d'acétates allyliques tertiaires, et plus particulièrement de l'acétate de linalyle à partir du bromhydrate ou du chlorhydrate de myrcène, l'acétate de linalyle étant d'un intérêt considérable en tant que tel (parfumerie), ou pour la synthèse des vitamines A et E et de produits ayant une structure terpénique.

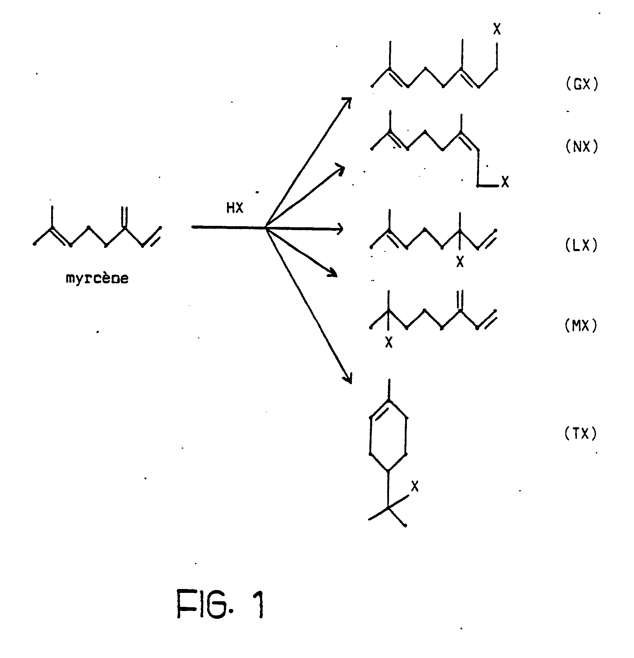

L'hydrohalogénation du myrcène peut conduire à un mélange d'halogénures, appelé communément bromhydrate ou chlorhydrate de myrcène, selon le schéma représenté figure 1 dans lequel

X représente un atome de brome ou de chlore,

GX est l'halogénure de géranyle

NX est l'halogénure de néryle

LX est l'halogénure de linalyle

MX est l'halogénure de myrcényle, et

TX est l'halogénure de terpényle.

Il est particulièrement intéressant de pouvoir obtenir, sélectivement et avec de bons rendements, l'acétate de linalyle à partir d'un tel mélange.

Selon le brevet américain US 2 794 826, le chlorhydrate de myrcène, obtenu par action de l'acide chlorhydrique sur le myrcène, traité par l'iodure cuivreux ou par le chlorure cuivreux en présence d'iodure de potassium et par l'acétate de potassium à 120 °C dans l'anhydride acétique conduit à un mélange contenant 43 % d'acétate de linalyle et 9 % du mélange des acétates de géranyle et de néryle.

D'après le brevet américain US 3 076 839, l'hydrochloration du myrcène en présence de chlorure cuivreux à 10-20 °C conduit à un mélange (contenant 75 à 80 % du mélange des chlorures de géranyle et de néryle, 5 à 10 % de chlorure de linalyle et 10 à 15 % de chlorure de terpényle) qui, traité par l'acétate de sodium en présence de chlorure cuivreux dans l'acide acétique à 25-30 °C, conduit à un mélange contenant 75 à 80 % d'acétate de linalyle, 5 à 10 % d'acétates de géranyle et de néryle et 8 à 10 % de chlorure de terpényle.

D'après le brevet allemand DE 1 274 117 le chlorhydrate de myrcène est transformé en acétate de linalyle avec un rendement de 64 % en opérant en présence d'acétate cuivrique, d'acétate de sodium et de carbonate de calcium, la sélectivité étant voisine de 95 %.

D'après le brevet américain US 3 031 442, le chlorhydrate de myrcène (contenant 75 à 80 % de chlorures de géranyle et de néryle, 5 à 10 % de chlorure de linalyle et 10 à 15 % de chlorure de terpényle) traité par l'acétate de sodium en présence de triéthylamine à 85-90 °C conduit à un mélange contenant 75 à 80 % d'acétates de géranyle et de néryle, 8 à 10 % d'acétate de linalyle et 8 à 10 % de chlorure de terpényle.

Ainsi d'après les procédés connus, l'acétate de linalyle ne peut être préparé qu'avec des rendements et une sélectivité médiocres à partir d'un halohydrate de myrcène.

Il a maintenant été trouvé, et c'est ce qui fait l'objet de la présente invention, qu'un ester allylique tertiaire éventuellement halogéné, de préférence l'acétate, peut être obtenu sélectivement et avec de bons rendements en traitant un halogénure allylique primaire et/ou tertiaire éventuellement halogéné ou leurs mélanges par un sel de métal alcalin d'un acide carboxylique, de préférence l'acide acétique, en présence d'un sel cuivreux tel qu'un halogénure cuivreux (iodure, chlorure) en présence d'un sel d'ammonium quaternaire ou d'un sel de phosphonium choisi parmi les halogénures de tétraalcoylammonium, les halohydrates de trialcoylamines et les halogénures de tétraalcoylphosphonium à une température comprise entre — 20 et 50 °C.

Généralement, pour la mise en oeuvre du procédé selon l'invention, on utilise un halogénure cuivreux dans un rapport molaire compris entre 0,05 et 10 %, de préférence entre 0,1 et 3 % par rapport à l'halogénure allylique mis en oeuvre associé à un sel d'ammonium quaternaire ou à un sel de phosphonium dans un rapport molaire compris entre 0,05 et 10 %, de préférence entre 0,1 et 3 % par rapport à l'halogénure allylique mis en oeuvre.

Comme sels d'ammonium quaternaire ou sels de phosphonium qui conviennent particulièrement bien peuvent être cités les chlorures et bromures de tétra-n. butylammonium ou de tétra-n. octylammonium, le chlorhydrate de triéthylamine et le chlorure de tétra-n. butylphosphonium.

Il est particulièrement avantageux d'opérer dans un solvant organique inerte dans lequel le complexe catalytique peut être solubilisé. Comme solvants peuvent être utilisés des hydrocarbures aliphatiques halogénés tels que le chlorure de méthylène, des acides organiques tels que l'acide acétique, les anhydrides d'acides organiques tels que l'anhydride acétique, les hydrocarbures aliphatiques tels que l'hexane, cycloaliphatiques tels que le cyclohexane ou aromatiques tels que le benzène ou leurs mélanges.

Le sel de métal alcalin de l'acide carboxylique est utilisé en quantité de préférence stoechiométrique par rapport à l'halogénure allylique mis en oeuvre. Comme sels de métal alcalin conviennent particulièrement bien les sels de sodium et de potassium.

Les halogénures allyliques primaires et/ou tertiaires éventuellement halogénés et leurs mélanges utilisés pour la mise en oeuvre du procédé selon l'invention peuvent être obtenus par hydro-

halogénation de polyènes contenant deux doubles liaisons conjuguées terminales et une ou plusieurs doubles liaisons dans la chaîne hydrocarbonée au moyen d'un hydracide halogéné tel que l'acide chlorhydrique ou l'acide bromhydrique en présence d'un catalyseur constitué d'un halogénure cuivreux tel que le chlorure ou l'iodure cuivreux et d'un sel d'ammonium quaternaire ou d'un sel de phosphonium.

L'hydrohalogénation d'un polyène, contenant deux doubles liaisons conjuguées terminales et une ou plusieurs doubles liaisons dans la chaîne hydrocarbonée, au moyen d'un hydracide halogéné, de préférence l'acide chlorhydrique, conduit, selon la quantité d'hydracide halogéné utilisé, soit à un halogénure primaire et/ou tertiaire soit à un halogénure primaire et/ou tertiaire halogéné. Lorsque l'on utilise une quantité d'hydracide halogéné voisine de la stoechiométrie, l'hydrohalogénation s'effectue sélectivement sur la double liaison conjuguée terminale. Lorsque l'on utilise un excès d'hydracide halogéné déterminé en fonction du nombre total de doubles liaisons contenues dans le polyène, l'hydrohalogénation s'effectue sur la double liaison conjuguée terminale et sur les doubles liaisons présentes dans le polyène.

Généralement on utilise un halogénure cuivreux dans un rapport molaire compris entre 0,05 et 10 % par rapport au diène conjugué mis en oeuvre et le sel d'ammonium quaternaire ou le sel de phosphonium dans un rapport molaire compris entre 0, 05 et 10 % par rapport au diène conjugué mis en oeuvre.

Les sels d'ammonium quaternaire et les sels de phosphonium qui conviennent particulièrement bien sont les halogénures de tétraalcoylammonium (tétra-n. butylammonium, tétra-n. octylammonium), les halohydrates de trialcoylamines (chlorhydrate de triéthylamine) et les halogénures de tétraalcoylphosphonium (chlorure de tétra-n. butylphosphonium).

Généralement on opère dans un solvant organique choisi parmi les hydrocarbures aliphatiques halogénés (chlorure de méthylène), les acides organiques (acide acétique), les anhydrides d'acides organiques (anhydrique acétique), les hydrocarbures aliphatiques (hexane), cycloaliphatiques (cyclohexane), aromatiques (benzène).

La réaction est mise en oeuvre à une température inférieure à 20 °C et, de préférence inférieure à 0 °C.

Pour la mise en oeuvre du procédé selon l'invention conviennent particulièrement bien les halogénures allyliques primaires et/ou tertiaires ou leurs mélanges provenant de l'hydrohalogénation du myrcène, du β-farnésène, du β-springène du méthylène-3 triméthyl-7, 11, 15 hexadécadiène-1,6 (phytatriène), du méthylène-3 triméthyl-7, 11, 15 hexadécatriène-1,6, 15 (phytatétraène).

Le système catalytique utilisé pour la préparation des esters allyliques tertiaires selon la présente invention étant identique à celui utilisé pour la préparation des halogénures allyliques primaires et/ou tertiaires, le procédé selon l'invention peut être mis en oeuvre directement à partir des diènes conjugués terminaux sans isoler les halogénures allyliques intermédiaires.  -

Dans ces conditions, il est avantageux d'effectuer l'hydrohalogénation du polyène contenant une double liaison conjuguée terminale puis d'ajouter le mélange réactionnel contenant les halogénures allyliques primaires et/ou tertiaires éventuellement halogénés ou leurs mélanges au sel de métal alcalin de l'acide carboxylique associé à l'halogénure cuivreux et au sel d'ammonium quaternaire ou au sel de phosphonium. Il est également possible d'ajouter le sel de métal alcalin de l'acide carboxylique associé à l'halogénure cuivreux et au sel d'ammonium quaternaire ou au sel de phosphonium au mélange réactionnel provenant de l'étape d'hydrohalogénation du diène conjugué terminal.

Le procédé selon la présente invention permet d'obtenir les esters allyliques tertiaires avec des rendements supérieurs à 80 %, la sélectivité étant supérieure à 96 %.

Les esters allyliques tertiaires obtenus selon la présente invention peuvent être utilisés en tant que tels (parfumerie) ou comme intermédiaires dans la synthèse des vitamines A ou E ou de dérivés terpéniques.

Les exemples suivants, donnés à titre non limitatif, montrent comment l'invention peut être mise en pratique.

Exemple 1

Dans un réacteur A muni d'une agitation magnétique on introduit, sous atmosphère d'argon, 330 cm³ de chlorure de méthylène, 3,33 g de chlorhydrate de triéthylamine, 2,4 g de chlorure cuivreux et 164,9 g de myrcène technique titrant 69,6 %.

Le mélange réactionnel homogène, de couleur orangée, est refroidi à une température voisine de — 5 °C. On introduit alors en 5 heures, 43 g d'acide chlorhydrique sec.

La solution obtenue est alors ajoutée à 20 °C, en quelques minutes, dans un réacteur B, muni d'une agitation magnétique et placé sous atmosphère d'argon et contenant 145 g d'acétate de sodium anhydre, 2,4 g de chlorure cuivreux et 3, 33 g de chlorhydrate de triéthylamine. La température du réacteur B monte à 31 °C après 3 heures. On ajoute alors 2,4 g de chlorure cuivreux puis on pour suit l'agitation pendant 18 heures à 20 °C.

Le mélange réactionnel est alors versé sur 400 g de glace. On obtient ainsi une phase aqueuse d'un bleu intense et une phase organique brun clair. Après décantation, la phase organique est lavée par 300 cm³ d'une solution aqueuse de chlorure d'ammonium à 100 g/litre puis par 2 fois 200 cm³ d'eau et enfin séchée sur carbonate de potassium. Après filtration et évaporation du solvant, on obtient 237,1 g d'une huile contenant 59 % d'acétate de linalyle. Le rendement est de 84,5 % par rapport au myrcène pur présent dans le produit technique.

La sélectivité, exprimée par le rapport acétate de linalyle/acétate de linalyle + acétate de géranyle + acétate de néryle, est de 97 %.

Exemple 2

On ajoute rapidement 20 g de chlorhydrate de
myrcène à un mélange de 19 g d'acétate de
sodium anhydre, 0,64 g de chlorhydrate de triéthylamine et 0,46 g de chlorure cuivreux dans 4 cm³
de chlorure de méthylène à une température de
30 °C. Après 4 heures d'agitation à 30 °C, le
mélange réactionnel est hydrolysé. Après traitement du mélange réactionnel dans les conditions
décrites dans l'exemple 1, on obtient l'acétate de
linalyle avec un rendement de 70 % par rapport
au chlorhydrate de myrcène. La sélectivité est de
97 %.

Le chlorhydrate de myrcène est obtenu de la
manière suivante :

Dans un ballon tricol de 250 cm³ muni d'un
agitateur magnétique, d'un thermomètre, d'un
tube plongeant au niveau de l'agitateur et d'une
tête à hydrogéner, on introduit, sous atmosphère
d'argon, 1,4 g de chlorhydrate de triéthylamine et
120 cm³ de chlorure de méthylène. On ajoute
ensuite 1 g de chlorure cuivreux. On agite jusqu'à
l'obtention d'une solution jaune homogène qui
est refroidie à —5°. On ajoute 56 g de myrcène
dont la pureté est supérieure à 95 % puis 15 g
d'acide chlorhydrique anhydre en 5 heures.

Le mélange réactionnel est versé dans 200 cm³
d'une solution aqueuse de chlorure d'ammonium
à 100 g/litre. La phase organique est séparée par
décantation puis on extrait la phase aqueuse par
100 cm³ de chlorure de méthylène. Les phases
organiques réunies sont lavées par 3 fois 50 cm³
d'eau puis séchées sur carbonate de potassium.
Après filtration et évaporation du solvant, on
obtient 68,8 g de chlorhydrate de myrcène sous
forme d'une huile dont la composition est la
suivante :
- chlorures de géranyle et de néryle 88,1 %
- chlorure de linalyle 5,1 %
- chlorure de terpényle 1,9 %
- myrcène 3,7 %
- hydrocarbures en $C_{10}$ hydrochlorés 0,2 %
- hydrocarbures en $C_{10}$ dihydrochlorés 0,4 %
- hydrocarbures en $C_{10}$ (en dehors du myrcène)
0,6 %

Le taux de transformation du myrcène est de
97 % et le rendement en chlorures de géranyle,
néryle et linalyle isolés est de 88 %.

Exemple 3

On opère comme dans l'exemple 2 mais en
absence de chlorhydrate de triéthylamine. Après
4 heures d'agitation à 30 °C et traitement du
mélange réactionnel, on obtient l'acétate de linalyle avec un rendement de 30 %.
La sélectivité est de 98 %.

Exemple 4

Dans un tricol de 250 cm³, on introduit, sous
atmosphère d'argon, 221 mg de chlorure cuivreux, 6,1 g d'acétate de sodium sec et 274 mg de
chlorhydrate de triéthylamine dans 30 cm³ de
chlorure de méthylène. On ajoute 15 g de tétrachloro-1,7,11,15 tétraméthyl-3,7,11,15 hexadécène-
2 en solution dans 20 cm³ de chlorure de méthylène. On maintient l'agitation pendant 6 heures.
Après hydrolyse à l'eau, on extrait le mélange
réactionnel par du pentane. Les phases organiques sont séchées puis concentrées à sec. On
obtient ainsi 13,85 g d'une huile constituée essentiellement d'acétoxy-3 trichloro-7,11,15 tétraméthyl-3,7,11,15 hexadécène-1.

La structure du produit obtenu est confirmée
par le spectre de masse et par le spectre de
résonance magnétique nucléaire du proton.

Le tétrachloro-1,7, 11, 15 tétraméthyl-3,7,11,15
hexadécène-2 peut être préparé de la manière
suivante :

Dans un réacteur de 250 cm³, on introduit, sous
atmosphère d'argon, 0,48 g de chlorhydrate de
triéthylamine, 15 cm³ de chlorure de méthylène,
10 cm³ d'acide acétique et 90 mg de chlorure
cuivreux. On agite le mélange réactionnel jusqu'à
l'obtention d'une solution homogène. On refroidit
à — 10 °C puis on ajoute 10 g de β-springène et, en
1 heure, 5,2 g d'acide chlorhydrique gazeux sec.
Après traitement du mélange réactionnel, on
obtient 14,2 g de tétrachloro-1,7,11,15 tétraméthyl-3,7,11,15 hexadécène-2 avec un rendement de 94 %.

Exemple 5

Dans un tricol de 100 cm³, on introduit, sous
atmosphère d'argon, 250 mg de chlorure cuivreux
et 8,1 g d'acétate de sodium sec. On ajoute
ensuite 25 cm³ de chlorure de méthylène contenant 340 mg de chlorhydrate de triéthylamine
puis 17,45 g d'un mélange de dichloro-3,7 tétraméthyl-3,7,11,15 hexadécène-1 et de dichloro-1,7
tétraméthyl-3,7,11,15 hexadécène-2 dans 25 cm³
de chlorure de méthylène.

La température du mélange réactionnel passe
de 20 a 32 °C en 15 minutes après la fin de
l'addition du composé dichloré puis redescend à
une température voisine de 20 °C. On agite pendant 6 heures. On ajoute 100 cm³ d'eau puis
extrait le mélange réactionnel par 2 fois 100 cm³
de pentane. Les phases organiques sont séchées,
filtrées puis concentrées à sec. On obtient ainsi
16,8 g d'huile constituée essentiellement
d'acétoxy-3 chloro-7 tétraméthyl-3,7,11,15 hexadécène-1.

La structure du produit obtenu est confirmée
par le spectre de masse et les spectres de résonance magnétique nucléaire du proton et du $^{13}$C.

Le mélange de dichloro-3,7 tétraméthyl-
3,7,11,15 hexadécène-1 et de dichloro-1,7 tétraméthyl-3,7,11,15 hexadécène-2 peut être préparé
de la manière suivante :

Dans un tricol de 250 cm³, on introduit, sous
atmosphère d'argon, 360,5 mg de chlorhydrate de
triéthylamine (0,26 x 10 mole), 126 mg de chlorure
cuivreux (0,13 x 10⁻² mole), 9 cm³ d'acide

# 0 179 685

acétique et 9 cm³ de chlorure de méthylène. On agite jusqu'à l'obtention d'une solution homogène jaune. On refroidit à 0 °C puis on ajoute rapidement 13,96 g de méthylène-3 triméthyl-7,11,15 hexadécadiène-1,6 dont la pureté est de 95 %. On refroidit la solution à une température voisine de — 5 °C puis on fait passer un courant d'acide chlorhydrique gazeux anhydre pendant 1 heure 20 minutes de façon à introduire 5 g (0,137 mole) d'acide chlorhydrique. Après 30 minutes d'agitation à une température voisine de — 5°C, on verse le mélange réactionnel dans 20 cm³ de pentane et 20 cm³ d'une solution aqueuse de chlorure d'ammonium à 10 % en poids à une température voisine de 20°C. La phase organique est séparée par décantation puis séchée sur sulfate de sodium. Après filtration et évaporation du solvant, on obtient 17,31 g d'un produit brut dont l'analyse par spectrographie de masse et par résonance magnétique nucléaire du proton révèle la présence de 90 % d'un mélange de dichloro-1,7 tétraméthyl-3,7,11,15 hexadécène-2 et de dichloro-3,7 tétraméthyl-3,7,11,15 hexadécène-1.

Pour vérifier la linéarité du squelette du produit obtenu on traite 1,7 g de l'huile constituée essentiellement d'acétoxy-3 chloro-7 tétraméthyl-3,7,11,15 hexadécène-1 en solution dans 20 cm³ d'éthanol, à 80 °C sous une pression de 20 bars d'hydrogène en présence de 170 mg de palladium sur noir à 10 %. Après filtration du catalyseur et évaporation du solvant, le dosage par chromatographie en phase vapeur avec étalon interne montre que le rendement en phytane est de 83,7 % par rapport au triène mis en oeuvre.

La sélectivité en phytane par rapport aux autres isomères est de 98 %.

## Revendications

1. Procédé de préparation d'un ester allylique tertiaire éventuellement halogéné par action d'un sel de métal alcalin d'un acide carboxylique sur un halogénure allylique primaire et/ou tertiaire éventuellement halogéné et leurs mélanges, préparé éventuellement in situ par hydrohalogénation, au moyen d'un hydracide halogéné; d'un polyène contenant deux doubles liaisons conjuguées terminales et éventuellement une ou plusieurs doubles liaisons dans la chaîne hydrocarbonée, en présence d'un catalyseur constitué d'un sel cuivreux et d'un sel d'ammonium quaternaire ou d'un sel de phosphonium, caractérisé en ce que l'on opère en présence d'un catalyseur constitué d'un halogénure cuivreux tel que le chlorure ou l'iodure cuivreux associé à un sel d'ammonium quaternaire ou à un sel de phosphonium choisi parmi les halogénures de tétraalcoylammonium, les halohydrates de trialcoylamines et les halogénures de tétraalcoylphosphonium, à une température comprise entre — 20 et 50 °C.

2. Procédé selon la revendication 1 caractérisé en ce que l'ester allylique tertiaire est l'acétate.

3. Procédé selon l'une des revendications 1 ou 2 caractérisé en ce que l'halogénure cuivreux est utilisé dans un rapport molaire compris entre 0,05 et 10 % par rapport à l'halogénure allylique primaire et/ou tertiaire éventuellement halogéné ou ses mélanges.

4. Procédé selon l'une des revendications 1 ou 2 caractérisé en ce que le sel d'ammonium quaternaire ou le sel de phosphonium est utilisé dans un rapport molaire compris entre 0,05 et 10 % par rapport à l'halogénure allylique primaire et/ou tertiaire éventuellement halogéné ou ses mélanges.

5. Procédé selon la revendication 1 caractérisé en ce que le sel d'ammonium quaternaire ou le sel de phosphonium est choisi parmi les chlorures et bromures de tétra-n. butylammonium et tétra-n. octylammonium, le chlorhydrate de triéthylamine et le chlorure de tétra-n. butylphosphonium.

6. Procédé selon la revendication 1 caractérisé en ce que l'on opère dans un solvant organique inerte dans lequel le complexe catalytique est soluble.

7. Procédé selon la revendication 6 caractérisé en ce que le solvant est choisi parmi les hydrocarbures aliphatiques halogénés, les acides organiques, les anhydrides d'acides organiques, les hydrocarbures aliphatiques, cycloaliphatiques ou aromatiques et leurs mélanges.

8. Procédé selon la revendication 1 caractérisé en ce que l'halogénure allylique primaire et/ou tertiaire et ses mélanges provient de l'hydrohalogénation du myrcène, du β-farnésène ou du β-springène, du phytatriène ou du phytatétraène.

## Claims

1. Process for the preparation of an optionally halogenated tertiary allyl ester by reaction of an alkali metal salt of a carboxylic acid with an optionally halogenated primary and/or tertiary allyl halide or mixtures thereof, optionally prepared in situ by hydrohalogenation, by means of a halogen hydracid, of a polyene containing two terminal conjugated double bonds and optionally one or more double bonds in the hydrocarbon chain, in the presence of a catalyst consisting of a cuprous salt and a quaternary ammonium salt or phosphonium salt, characterised in that the said allyl ester is prepared in the presence of a catalyst consisting of a cuprous halide, such as cuprous chloride or iodide, together with a quaternary ammonium salt or a phosphonium salt chosen from among the tetraalkylammonium halides, the trialkylamine hydrohalides and the tetraalkylphosphonium halides, at a temperature of between — 20 and 50 °C.

2. Process according to Claim 1, characterised in that the tertiary allyl ester is an acetate.

3. Process according to either of Claims 1 and 2, characterised in that the cuprous halide is used in a molar ratio of between 0.05 and 10 % relative to the optionally halogenated primary and/or tertiary allyl halide or the mixtures thereof.

4. Process according to either of claims 1 and 2, characterised in that the quaternary ammonium salt or the phosphonium salt is used in a molar ratio of between 0.05 and 10 % relative to the optionally halogenated primary and/ or tertiary allyl halide or the mixtures thereof.

5. Process according to Claim 1, characterised in that the quaternary ammonium salt or phosphonium salt is chosen from among tetra-n-butylammonium and tetra-n-octylammonium chloride and bromide, triethylamine hydrochloride and tetra-n-butylphosphonium chloride.

6. Process according to Claim 1, characterised in that it is carried out in an inert organic solvent in which the catalyst complex is soluble.

7. Process according to Claim 6, characterised in that the solvent is chosen from among halogenated aliphatic hydrocarbons, organic acids, organic acid anhydrides, aliphatic, cycloaliphatic or aromatic hydrocarbons and mixtures of these.

8. Process according to Claim 1, characterised in that the primary and/or tertiary allyl halide and the mixtures thereof originate from the hydrohalogenation of myrcene, of ß-farnesene, of ß-springene, of phytatriene or of phytatetraene.

**Patentansprüche**

1. Verfahren zur Herstellung eines gegebenenfalls halogenierten tertiären Allylesters durch Einwirkung eines Alkalimetallsalzes einer Carbonsäure auf ein gegebenenfalls halogeniertes primäres und/oder tertiäres Allylhalogenid und deren Mischungen, die gegebenenfalls in situ durch Hydrohalogenierung eines zwei endständige konjugierte Doppelbindungen und gegebenenfalls eine oder mehrere Doppelbindungen in der Kohlenwasserstoffkette enthaltenden Polyens mittels einer Halogengenwasserstoffsäure hergestellt wurden, in Gegenwart eines Katalysators, bestehend aus einem Kupfer (1) salz und einem quaternären Ammoniumsalz oder einem Phosphoniumsalz, dadurch gekennzeichnet, daß man in Gegenwart eines Katalysators, der aus einem Kupfer (I) halogenid, z. B. Kupferchlorid oder -jodid, in Kombination mit einem quaternären Ammoniumsalz oder einem Phosphoniumsalz, ausgewählt aus den Tetraalkylammoniumhalogeniden, den Trialkylaminhalogenhydraten und den Tetraalkylphosphoniumhalogeniden, besteht, bei einer Temperatur zwischen — 20 und 50 °C arbeitet.

2. Verfahren nach Anspruch 1, dadurch gekennzeichnet, daß der tertiäre Allylester das Acetat ist.

3. Verfahren nach einem der Ansprüche 1 oder 2, dadurch gekennzeichnet, daß das Kupfer (I) halogenid in einem molaren Verhältnis von 0,05 bis 10 %, bezogen auf das gegebenenfalls halogenierte primäre und/oder tertiäre Allylhalogenid oder dessen Mischungen, eingesetzt wird.

4. Verfahren nach einem der Ansprüche 1 oder 2, dadurch gekennzeichnet, daß das quaternäre Ammoniumsalz oder das Phosphoniumsalz in einem molaren Verhältnis von 0,05 bis 10 %, bezogen auf das gegebenenfalls halogenierte primäre und/oder tertiäre Allylhalogenid oder dessen Mischungen, eingesetzt wird.

5. Verfahren nach Anspruch 1, dadurch gekennzeichnet, daß das quaternäre Ammoniumsalz oder das Phosphoniumsalz ausgewählt wird aus Tetra-n. butylammonium- und Tetra-n. octylammoniumchlorid und -bromid/ Triathylaminchlorhydrat und Tetra-n. butylphosphoniumchlorid.

6. Verfahren nach Anspruch 1, dadurch gekennzeichnet, daß man in einem inerten organischen Lösungsmittel arbeitet, in dem der katalytische Komplex löslich ist.

7. Verfahren nach Anspruch 6, dadurch gekennzeichnet, daß das Lösungsmittel ausgewählt wird aus den halogenierten aliphatischen Kohlenwasserstoffen, den organischen Säuren, den organischen Säureanhydriden, den aliphatischen, cycloaliphatischen oder aromatischen Kohlenwasserstoffen und ihren Mischungen.

8. Verfahren nach Anspruch 1, dadurch gekennzeichnet, daß das primäre und/oder tertiäre Allylhalogenid und seine Mischungen aus der Hydrohalogenierung von Myrcen, β-Farnesen oder β-Springen, Phytatrien oder Phytatetraen stammen.

FIG. 1